# EUROPEAN PATENT SPECIFICATION

(11) **EP 2 002 809 B1**
(45) Date of publication and mention of the grant of the patent: **13.04.2011**
(21) Application number: 08075768.5
(22) Date of filing: 12.08.1999
(51) Int. Cl.: A61F 5/00

(54) **Food intake restriction device**
Vorrichtung zur Begrenzung der Nahrungsaufnahme
Dispositif de restriction d'ingestion de nourriture

(30) Priority: 13.08.1998 US 133319
(43) Date of publication of application: 17.12.2008
(62) Divisional of application: 05075924.0
(73) Proprietor: Obtech Medical AG, 6341 Baar (CH)
(72) Inventor: Forsell, Peter, 6313 Menzingen (CH)
(74) Representative: Tunstall, Christopher Stephen

(56) References cited:
- EP-A- 0 876 808
- WO-A-96/01597
- FR-A- 2 756 485
- GB-A- 1 174 814
- US-A- 3 750 194

## Description

The present invention relates to a food intake restriction device for forming a stoma opening in the stomach or esophagus of a patient, as defined in claim 1, the device comprising an elongated restriction member, forming means for forming the elongated restriction member into at least a substantially closed loop around the patient's stomach or esophagus, said loop defining a restriction opening, and an adjustment means for adjusting the restriction member in said loop to change the size of said restriction opening. The term "patient" includes an animal or a human being.

Food intake restriction devices in the form of gastric banding devices, in which a band encircles a portion of a patient's stomach to restrict the food intake of the patient, have been used in surgery for morbid obesity to form a small gastric pouch above the band and a reduced stoma opening in the stomach. Although such a band is applied around the stomach to obtain an optimal stoma opening during surgery, some prior gastric banding devices are provided with an adjustment means enabling a minor post-operation adjustment of the size of the stoma opening. In all such prior art devices such as disclosed in U.S. Patent No. 4,592,339, European Patent No. 0611561 and International Patent Application WO 94/27504, the adjustment means typically comprises an inflatable cavity in the band and an injection port in fluid connection with the inflatable cavity for adding fluid to or withdrawing fluid from the latter. In practice, the band is made of silicone rubber which is a material approved for implantation and the fluid is a liquid such as an isotonic salt solution.

It has also been found that the volume of the gastric pouch above the band increases in size up to ten times after operation. Therefore the pouch volume during surgery needs to be very small, approximately 7 ml. To enable the patient to feed the stomach with sufficient nutrition immediately after an operation considering such a small gastric pouch, the stoma initially needs to be relatively large and later needs to be substantially reduced, as the pouch volume increases. To be able to achieve a significant range of adjustment of the band, the cavity in the band has to be relatively large and is defined by a thin flexible wall, normally made of silicone material. Furthermore, the size of the stoma opening has to be gradually reduced during the first year after surgery as the gastric pouch increases in size. As indicated above, the reduction of the stoma opening using the prior art devices is achieved by adding liquid to the cavity of the band via the injection port to expand the band radially inwardly.

A great disadvantage of repeatedly injecting liquid via the injection port is the increased risk of the patient getting an infection in the body area surrounding the injection port. If such an infection occurs the injection port has to be surgically removed from the patient. Moreover, such an infection might be spread along the tube interconnecting the injection port and the band to the stomach, causing even more serious complications. Thus, the stomach might be infected where it is in contact with the band, which might result in the band migrating through the wall of the stomach. Also, it is uncomfortable for the patient when the necessary, often many, post-operation adjustments of the stoma opening are carried out using an injection needle penetrating the skin of the patient into the injection port.

It may happen that the patient swallows pieces of food too large to pass through the restricted stoma opening. If that occurs the patient has to visit a doctor who can remove the food pieces, if the band design so permits, by withdrawing some liquid from the band to enlarge the stoma opening to allow the food pieces to pass the stoma. Then, the doctor has to add liquid to the band in order to regain the restricted stoma opening. Again, these measures require the use of an injection needle penetrating the skin of the patient, which is uncomfortable for the patient.

European patent application EP 0 876 808, which qualifies as prior art under Art 54(3) EPC, discloses a device which includes an adjustable strap for implanting around the stomach. The strap has a variable volume cavity filled with a liquid. The volume of liquid in teh cavity is adjusted by a system which includes a control box that is connected to the cavity and implanted in the patient's body. The control box contains a battery, an electronic control unit and an electronically driven pump.

FR 2 756 485 discloses a prosthetic urinary sphincter with an external control. The sphincter includes a ring-shaped balloon which is arranged inside an inextensible sheath enclosing the corporeal duct. A pump positioned within a fluid circuit is controlled by electromagnetic induction by means of an antenna located inside the body.

WO 96/01597 discloses a vessel occlusive prosthesis for occluding a fluid conveying vessel. The prosthesis includes an elongated member at least partially encircling the vessel, and means connected to the elongated member for applying tension to the elongated member.

US 3,750,194 discloses a device for closing a natural or implanted body passage which uses an implantable fluid reservoir and a distensible member that is adapted to be connected to the passage. An implantable pump is connected to the fluid reservoir and the distensible member for pumping fluid from the reservoir to the distensible member

GB 1,174,814 discloses a device for occluding ducts. The device is operated either manually or by facilities located outside of the body

An object of the invention is to provide a food intake restriction device for forming a stoma opening in the stomach or esophagus of a patient which permits regular post-operation adjustments that are comfortable for the patient.

Another object of the present invention is to provide a food intake restriction device for forming a stoma opening in the stomach or esophagus of a patient which is easy to adjust and does not require the use of an injection needle for accomplishing post-operation adjustments of the stoma opening.

These objects are obtained by a food intake restriction device as recited in the claims.

The remote control means may advantageously be capable of obtaining information on the size of the restriction opening and to command the adjustment means to adjust the restriction member in response to obtained information.

An implantable motor may suitably be provided for operating the adjustment means and said means for wireless transfer of energy may be adapted to directly power the motor with transferred energy. The energy transferred by said means for transfer of energy may comprise wave signals, an electric field or a magnetic field.

Preferably, the wireless remote control means comprises separate signal transmitting means and implantable signal receiving means. The signal receiving means comprises a control unit adapted to control the adjustment means in response to signals from the signal transmitting means. For example, the signal transmitting and signal receiving means may be adapted to transmit and receive signals in the form of digital pulses, which may comprise a magnetic or electric field. Alternatively, which is preferred, the signal transmitting and signal receiving means may be adapted to transmit and receive wave signals, which may comprise electromagnetic waves, sound waves or carrier waves for remote control signals.

The food intake restriction device further comprises an implantable energizer unit for providing energy to energy consuming components of the device to be implanted in the patient, such as electronic circuits and/or a motor for operating the adjustment means. The control unit may be adapted to power such an implanted motor with energy provided by the energizer unit in response to signals received from the signal transmitting means. Any known or conventional signal transmitting or receiving device that is suitable for use with a human or mammal patient may be provided as the signal transmitting or receiving means. The signals may comprise electromagnetic waves, such as infrared light, visible light, laser light, micro waves, or sound waves, such as ultrasonic waves or infrasonic waves, or any other type of wave signals. The signals may also comprise electric or magnetic fields, or pulses. All of the above-mentioned signals may comprise digital signals.

The motor may be any type of motor, such as a pneumatic, hydraulic or electric motor and the energizer unit may be adapted to power the motor with pressurized gas or liquid, or electrical energy, depending on the type of motor. Where the motor is an electric motor, it may power pneumatic or hydraulic equipment.

In accordance with a first particular embodiment of the invention, the energizer unit comprises a power supply and the control unit is adapted to power the motor with energy from the power supply. Preferably, the power supply is an electric power supply, and the motor is an electric motor.

In accordance with a second, preferred, particular embodiment of the invention, the energizer unit is adapted to transfer energy from the signals, as they are transmitted to the signal receiving means, into electric energy for powering the implanted electronic components. For example, the energizer unit may be adapted to transfer the energy from the signals into direct or alternating current.

In case there is an implanted electric motor for operating the adjustment means the energizer unit may also power the motor with the transferred energy. Advantageously, the control unit is adapted to directly power the electric motor with electric energy, as the energizer unit transfers the signal energy into the electric energy. This embodiment is particularly simple and does not require any recurrent invasive measures for exchanging empty power supplies, such as batteries, that is required in the first embodiment described above.

To expand the field of application of the second preferred embodiment to adjustment means of the type that requires more, but still relatively low, power for its operation, the energizer unit may comprise a rechargeable electric power supply for storing the electric energy obtained and the control unit be adapted to power the electric motor with energy from the rechargeable electric power supply in response to signals received from the signal transmitting means. In an initial charging step the rechargeable power supply can be charged over a relatively long time (e.g. a few seconds up to a half hour) without powering the electric motor. In a following operating step, when the power supply has been charged with sufficient energy, the control unit powers the electric motor with energy from the charged power supply to operate the adjustment means, so that a desired change of the patient's stoma opening is achieved. If the capacity of the power supply is insignificant to achieve the necessary adjustment in one single operating step, the above steps may conveniently be repeated until the desired adjustment is achieved.

The electric power supply suitably comprises an inexpensive simple capacitor. In this case, the electric motor may be a stepping motor.

In connection with the second preferred embodiment the signal transmitting means may be adapted to transmit electromagnetic wave signals and the energizer unit be adapted to draw radiant energy from the electromagnetic wave signals, as they are transmitted to the signal receiving means, and transfer the radiant energy into electric energy.

In the above-described embodiments of the invention, the signal transmitting means may be adapted to transmit electromagnetic wave signals and the energizer unit be adapted to draw radiant energy from the electromagnetic wave signals, as they are transmitted to the signal receiving means, and to transfer the radiant energy into said current. The energizer unit suitably comprises a coil of the signal receiving means for inducing an alternating current as electromagnetic wave signals are transmitted through the coil and a rectifier for rectifying the alternating current. The rectified current is used for charging the rechargeable power source.

Alternatively, the signal transmitting and receiving means may solely be used for control signals and further signal transmitting and receiving means be provided for transferring signal energy to implanted components. By such a double system of signal transmitting and receiving means the advantage is obtained that the two systems can be designed optimally for their respective purposes, namely to transmit control signals and to transfer energy from signals.

Although the above-described embodiments of the invention may very well be implemented in connection with the prior types of food intake restriction devices discussed above, in which the adjustment means comprises an inflatable cavity of a restriction member, it is preferred to use an elongated restriction member which is non-inflatable, in order to avoid the risk of fluid leaking from the cavity. Furthermore, it is preferred to use an adjustment means which is designed to mechanically adjust the non-inflatable restriction member.

The invention is described in more detail in the following by way of example with reference to the accompanying drawings, in which
Figure 1 is a schematic cross-sectional view of a part of the food intake restriction device in accordance with the present invention;
Figures 2 and 3 are cross-sectional views taken along the lines II-II and III-III, respectively, of Fig. 1;
Figure 4 is a block diagram illustrating remote control components of the device of the invention;
Figure 5 is a schematic view of exemplary circuitry used for the components of the block diagram of Fig. 4.

Referring to the drawing figures, like reference numerals designate identical or corresponding elements throughout the several figures.

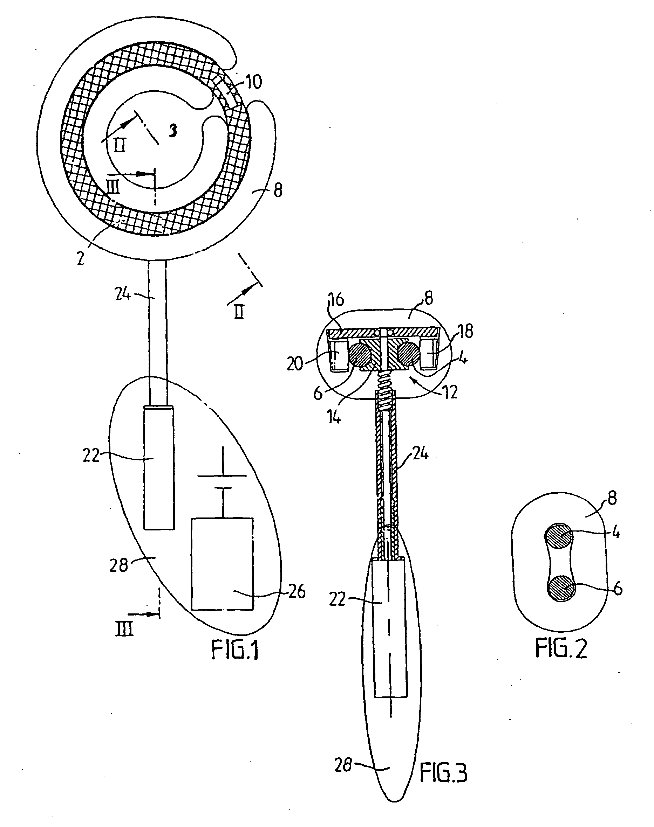

Figs. 1-3 show an example of a part of the food intake restriction device of the invention, comprising a circular resilient non-inflatable restriction member 2 with two overlapping end portions 4,6. The restriction member 2 defines a substantially circular restriction opening and is enclosed in an elastic soft hose 8 except at a releasable and lockable joint 10 of the restriction member 2, which when released enables application of the restriction member 2 with its hose 8 around the esophagus or stomach of a patient in a surgical procedure. All of the in body components are desirably of bio-compatible material or covered with bio-compatible material.

An adjustment means 12 mechanically adjusts the longitudinal extension of the restriction member 2 to change the size of said restriction opening. The adjustment means may comprise any known or conventional mechanical device for this purpose. The illustrated embodiment of the device 12 comprises a pulley 14 in frictional engagement with the overlapping end portions 4,6. The pulley 14 is journalled on a holder 16 placed in the hose 8 and provided with two counter pressure rollers 18,20 pressing the respective end portions 4, 6 against the pulley 14 to increase the frictional engagement therebetween. An electric motor 22 is connected to the pulley 14 via a long flexible drive shaft 24 and is moulded together with an energizer unit 26 in a body 28 of silicone. The length of the flexible drive shaft 24 is selected so that the body 28 can be placed in a desired position in the abdomen of the patient. All components are of bio-compatible material, or covered with bio-compatible material.

If the patient some time after the operation needs adjustment of the restriction opening of the restriction member 2, the energizer unit 26 is controlled to power the electric motor 22 either to rotate the pulley 14 in one direction to reduce the diameter of the circular restriction member 2 or to rotate the pulley 14 in the opposite direction to increase the diameter of the restriction member 2.

It should be understood that the implantable part of the device described above alternatively may be one of a variety of different adjustable designs. For example, the elongated restriction member may be inflatable by a fluid, which is pumped to and from the restriction member by a pump operated by the motor 22.

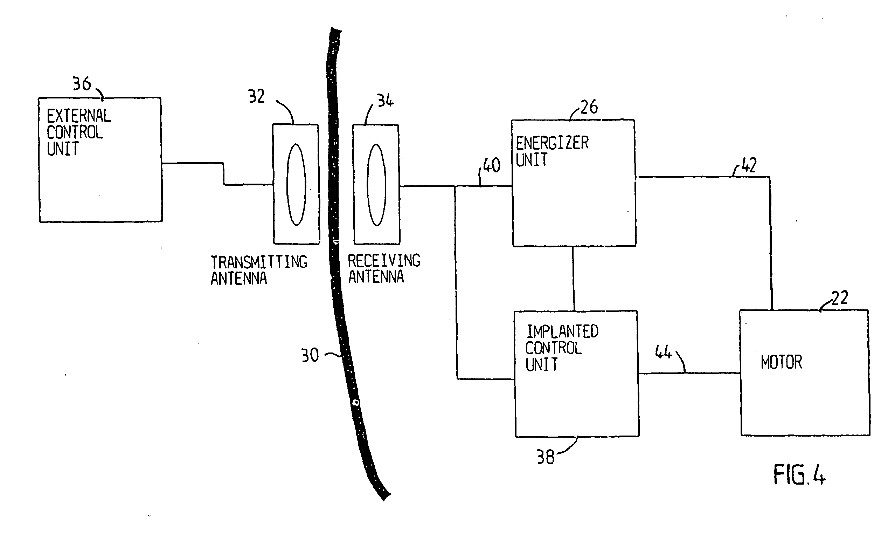

Fig. 4 shows the basic parts of an exemplary remote control system of the device of the invention including the electric motor 22. This remote control system is based on the transmission of electromagnetic wave signals, often of high frequencies on the order of 100 kHz - 1 gHz, through the skin 30 of the patient. For the first embodiment of the invention any known or developed remote control system may be utilized; electromagnetic wave signals do not need to be transmitted. In Fig. 4, all parts placed to the left of the skin 30 are located outside the patient's body and are thus not implanted, whereas all parts placed to the right of the skin 30 are implanted.

An external signal transmitting antenna 32 is to be positioned close to a signal receiving antenna 34 implanted close to the skin 30. As an alternative, the receiving antenna 34 may be placed, for example, inside the abdomen of the patient. The receiving antenna 34 may comprise a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 32 comprises a coil having about the same size as the coil of the receiving antenna 34 but wound with a thick wire that can handle the larger currents that is necessary. The coil of the transmitting antenna 32 is tuned to the same specific high frequency as the coil of the receiving antenna 34.

An external control unit 36 preferably comprises a microprocessor, a high frequency electromagnetic wave signal generator and a power amplifier. The microprocessor of the control unit 36 is adapted to switch the generator on/off and to modulate signals generated by the generator to send digital information via the power amplifier and the antennas 32,34 to an implanted control unit 38. To avoid accidental random high frequency fields triggering control commands, digital signal codes are used. A conventional keypad 37 placed on the external control unit 36 is connected to the microprocessor thereof. The keypad 37 is used to order the microprocessor to send digital signals to either increase or decrease the size of the restriction opening 3 defined by the loop of the restriction member 2. The microprocessor starts a command by applying a high frequency signal on the antenna 32. After a short time, when the signal has energized the implanted parts of the control system, commands are sent to increase or decrease the size of said restriction opening of the restriction member 2 in predefined steps. The commands are preferably sent as digital packets in the form illustrated below.

| Start pattern, | Command, | Count, | Checksum, |
|---|---|---|---|
| 8 bits | 8 bits | 8 bits | 8 bits |

The commands are sent continuously during a rather long time period, e.g. about 30 seconds or more. When a new increase or decrease step is desired the Count byte is increased by one to allow the implanted control unit 38 to decode and understand that another step is demanded by the external control unit 36. If any part of the digital packet is erroneous, its content is simply ignored.

Through a line 40, the implanted energizer unit 26 draws energy from the high frequency electromagnetic wave signals received by the receiving antenna 34. The energizer unit 26 stores the energy in a power supply, such as a large capacitor, powers the control unit 38 and powers the electric motor 22 via a line 42.

The control unit 38 comprises a demodulator and a microprocessor. The demodulator demodulates digital signals sent from the external control unit 36. The microprocessor of the control unit 38 receives the digital packet, decodes it and, provided that the power supply of the energizer unit 26 has sufficient energy stored, sends a signal via a signal line 44 to the motor 22 to either increase or decrease the size of the restriction opening 3 of the restriction member 2 depending on the received command code.

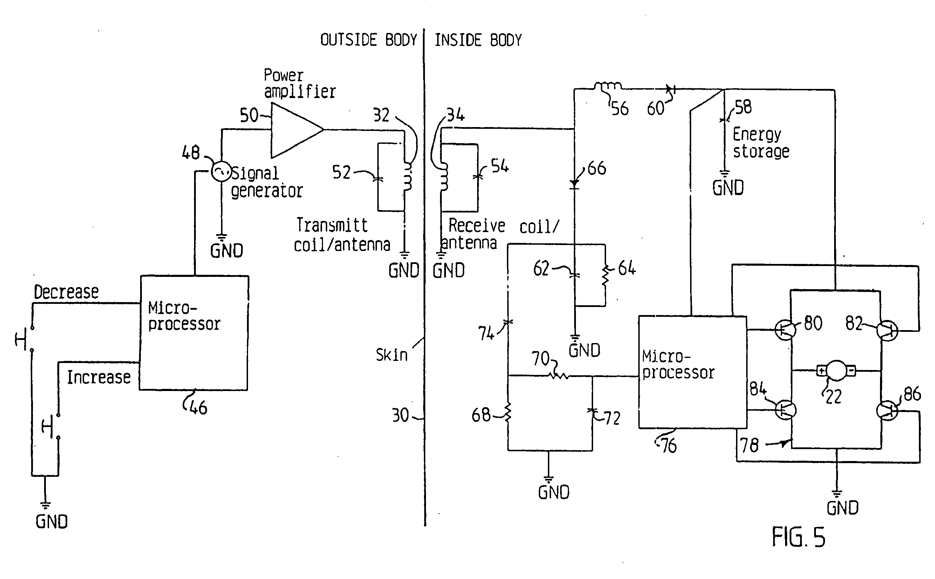

With reference to Fig. 5, the remote control system schematically described above will now be described in accordance with a more detailed embodiment. The external control unit 36 comprises a microprocessor 46, a signal generator 48 and a power amplifier 50 connected thereto. The microprocessor 46 switches the signal generator 48 on/off and to modulate signals generated by the signal generator 48 with digital commands that are sent to implanted components (to the right of skin 30 in Fig.5) of the implantable device. The power amplifier 50 amplifies the signals and sends them to the external signal transmitting antenna 32. The antenna 32 is connected in parallel with a capacitor 52 to form a resonant circuit tuned to the frequency generated by the signal generator 48.

The implanted signal receiving antenna coil 34 forms together with a capacitor 54 a resonant circuit that is tuned to the same frequency as the transmitting antenna 32. The signal receiving antenna coil 34 induces a current from the received high frequency electromagnetic waves and a rectifying diode 60 rectifies the induced current, which charges a storage capacitor 58. A coil 56 connected between the antenna coil 34 and the diode 60 prevents the capacitor 58 and the diode 60 from loading the circuit of the signal receiving antenna 34 at higher frequencies. Thus, the coil 56 makes it possible to charge the capacitor 58 and to transmit digital information using amplitude modulation.

A capacitor 62 and a resistor 64 connected in parallel and a diode 66 forms a detector used to detect amplitude modulated digital information. A filter circuit is formed by a resistor 68 connected in series with a resistor 70, in turn connected in series with a capacitor 72, in turn connected in series with the resistor 68 via ground, and a capacitor 74, one terminal of which is connected between the resistors 68,70 and the other terminal of which is connected between the diode 66 and the circuit formed by the capacitor 62 and resistor 64. The filter circuit is used to filter out undesired low and high frequencies. The detected and filtered signals are fed to an implanted microprocessor 76 that decodes the digital information and controls the motor 22 via an H-bridge 78 comprising transistors 80,82,84 and 86. The motor 22 can be driven in two opposite directions by the H-bridge 78.

The microprocessor 76 also monitors the amount of stored energy in the storage capacitor 58. Before sending signals to activate the motor 22, the microprocessor 76 checks whether the energy stored in the storage capacitor 58 is enough. If the stored energy is not enough to perform the requested operation, the microprocessor 76 waits for the received signals to charge the storage capacitor 58 before activating the motor 22.

With the aid of the remote control means it is possible to programme various sizes of the restriction opening which are to be set by the adjustment means depending on the time of the day. For example, the restriction opening may be relatively large at night, which may be beneficial with respect to the patient's well-being. Means may also be provided for sensing the actual size of the restriction opening so that the adjustment means can be controlled by the remote control means to adjust the restriction opening in response to such a sensing means.

There are a number of conceivable alternative embodiments of the invention that give the same result as the above-described embodiments. For example, the microprocessor of the external and implanted, respectively, control units may be replaced by discrete components. The power amplifier of the external control unit may be omitted if the signals generated by the signal generator are strong enough. Therefore, the invention is to be accorded the broadest interpretation of the appended claims to encompass all equivalent structures and assemblies.

One further advantage with this invention is that there may be a night button on the remote control setting the adjustment means in a position with a larger stoma diameter during the night, thus avoiding vomiting or nausa.

## Claims

1. A food intake restriction device for forming a stoma opening in the stomach or esophagus of a patient, the device comprising an elongated restriction member (2), forming means (10) for forming the elongated restriction member into at least a substantially closed loop around the patient's stomach or esophagus, said loop defining a restriction opening (3), and an implantable adjustment means (12) for adjusting the restriction member in said loop to change the size of said restriction opening, **characterised by** a wireless remote control means (22,26,32-44) for non-invasively controlling the adjustment means (12) to adjust the restriction member (2), to thereby obtain a desired size of said restriction opening, wherein:
the remote control means (22,26,32-44) comprises means for wireless transfer of energy from outside the patient's body to all energy consuming implantable components of the device.

2. The device according to claim 1, wherein the remote control means (22,26,32-44) comprises separate signal transmitting means (32,36) and implantable signal receiving means (34,38).

3. The device according to claim 2, wherein the signal receiving means (34,38) comprises a control unit (38) adapted to control the adjustment means (12) in response to signals from the signal transmitting means (32,36).

4. The device according to claim 3, further comprising an implantable energizer unit (26) for providing energy to energy consuming components of the device to be implanted in the patient.

5. The device according to claim 4, further comprising an implantable motor (22) for operating the adjustment means (12).

6. The device according to claim 5, wherein the control unit (38) is adapted to power the motor (22) with energy provided by the energizer unit (26) in response to signals received from the signal transmitting means (32,36).

7. The device according to claim 6, wherein the energizer unit (26) comprises a power supply (58).

8. The device according to claim 7, wherein the power supply (58) is an electric power supply and the motor (22) is an electric motor.

9. The device according to claim 8, wherein the electric power supply comprises a battery.

10. The device according to claim 1, further comprising an implantable motor (22) for operating the adjustment means (12), said means for wireless transfer of energy being adapted to directly power the motor with transferred energy.

11. The device according to claim 10, wherein the energy transferred by said means for transfer of energy comprises wave signals.

12. The device according to claim 10, wherein the energy transferred by said means for transfer of energy comprises an electric field or a magnetic field.

13. The device according to claim 1, wherein the remote control means (22,26,32-44) is capable of obtaining information on the size of the restriction opening(3) and to command the adjustment means (12) to adjust the restriction member (2) in response to obtained information.

## Patentansprüche

1. Restriktionseinheit für die Nahrungsaufnahme zum Bilden einer Stomaöffnung in dem Magen oder in der Speiseröhre eines Patienten, wobei die Einheit ein längliches Restriktionselement (2), eine Verformeinrichtung (10) zum Verformen des länglichen Restriktionselementes in wenigstens eine im Wesentlichen geschlossene Schleife um den Magen oder die Speiseröhre des Patienten, wobei die Schleife eine Restriktionsöffnung (3) definiert, und eine implantierbare Anpasseinrichtung (12) zum Anpassen des Restriktionselementes in der Schleife, um die Größe der Restriktionsöffnung zu ändern, aufweist, **gekennzeichnet durch** eine kabellose Fernsteuereinrichtung (22, 26, 32-44) zum nicht invasiven Steuern der Anpasseinrichtung (12), um das Restriktionselement (2) anzupassen, um somit eine gewünschte Größe der Restriktionsöffnung zu erhalten, wobei:
die Fernsteuereinrichtung (22, 26, 32-44) eine Einrichtung für die kabellose Übertragung von Energie von außerhalb des Körpers des Patienten zu allen Energie verbrauchenden implantierbaren Komponenten der Einheit aufweist.

2. Einheit nach Anspruch 1, bei der die Fernsteuereinrichtung (22, 26, 32-44) getrennte Signalsendeeinrichtungen (32, 36) und implantierte Signalempfangseinrichtungen (34, 38) aufweist.

3. Einheit nach Anspruch 2, bei der die Signalempfangseinrichtung (34, 38) eine Steuereinheit (38) aufweist, die dazu ausgelegt ist, die Anpasseinrichtung (12) als Antwort auf Signale von der Signalsendeeinrichtung (32, 36) zu steuern.

4. Einheit nach Anspruch 3, die weiter eine implantierbare Anregungseinheit (26) zum Bereitstellen von Energie für Energie verbrauchende Komponenten der Einheit, die in den Patienten implantiert werden sollen, aufweist.

5. Einheit nach Anspruch 4, die weiter einen implantierbaren Motor (22) zum Betreiben der Anpasseinrichtung (12) aufweist.

6. Einheit nach Anspruch 5, bei der die Steuereinheit (38) dazu ausgelegt ist, den Motor (22) mit Energie, die von der Anregungseinheit (26) als Antwort auf Signale, die von der Signalsendeeinrichtung (32, 36) empfangen werden, zu betreiben.

7. Einheit nach Anspruch 6, bei der die Anregeeinheit (26) eine Energiequelle (58) aufweist.

8. Einheit nach Anspruch 7, bei dem die Energiequelle (58) eine Quelle für elektrische Energie ist und der Motor (22) ein elektrischer Motor ist.

9. Einheit nach Anspruch 8, bei der die Quelle für elektrische Energie eine Batterie aufweist.

10. Einheit nach Anspruch 1, die weiter einen implantierbaren Motor (22) zum Betreiben der Anpasseinrichtung (12) aufweist, wobei die Einrichtung für die kabellose Übertragung von Energie so ausgelegt ist, dass sie den Motor direkt mit der übertragenden Energie betreibt.

11. Einheit nach Anspruch 10, bei der die Energie, die von der Einrichtung zum Übertragen von Energie übertragen wird, Wellensignale aufweist.

12. Einheit nach Anspruch 10, bei der die Energie, die von der Einrichtung zum Übertragen von Energie übertragen wird, ein elektrisches Feld oder ein Magnetfeld aufweist.

13. Einheit nach Anspruch 1, bei der die Fernsteuereinheit (22, 26, 32-44) in der Lage ist, Information über die Größe der Restriktionsöffnung (3) zu erhalten und der Anpasseinrichtung (12) zu befehlen, das Restriktionselement (2) als Antwort auf erhaltene Information anzupassen.

## Revendications

1. Dispositif de restriction d'ingestion de nourriture pour former un orifice de stomie dans l'estomac ou l'oesophage d'un patient, le dispositif comprenant un élément de restriction oblong (2), un moyen de formage (10) pour former l'élément de restriction oblong en au moins une boucle sensiblement fermée autour de l'estomac ou de l'oesophage du patient, ladite boucle définissant une ouverture de restriction (3), et un moyen d'ajustement implantable (12) pour ajuster l'élément de restriction dans ladite boucle afin de changer la taille de ladite ouverture de restriction, **caractérisé par** un moyen de commande à distance sans fil (22, 26, 32-44) pour commander de manière non invasive le moyen d'ajustement (12) pour ajuster l'élément de restriction (2) afin d'obtenir ainsi une taille souhaitée de ladite ouverture de restriction, où:
le moyen de commande à distance (22, 26, 32-44) comprend un moyen pour le transfert sans fil de l'énergie depuis l'extérieur du corps du patient à tous les composants implantables consommant de l'énergie du dispositif.

2. Dispositif selon la revendication 1, dans lequel le moyen de commande à distance (22, 26, 32-44) comprend un moyen de transmission de signaux séparé (32, 36) et un moyen de réception de signaux implantable (34, 38).

3. Dispositif selon la revendication 2, dans lequel le moyen de réception de signaux (34, 38) comprend une unité de commande (38) apte à commander le moyen d'ajustement (12) en réponse à des signaux du moyen de transmission de signaux (32, 36).

4. Dispositif selon la revendication 3, comprenant en outre une unité d'excitation implantable (26) pour fournir de l'énergie à des composants consommant de l'énergie du dispositif à implanter dans le patient.

5. Dispositif selon la revendication 4, comprenant en outre un moteur implantable (22) pour le fonctionnement du moyen d'ajustement (12).

6. Dispositif selon la revendication 5, dans lequel l'unité de commande (38) est apte à alimenter le moteur (22) en énergie fournie par l'unité d'excitation (26) en réponse à des signaux reçus du moyen de transmission de signaux (32, 36).

7. Dispositif selon la revendication 6, dans lequel l'unité d'excitation (26) comprend une alimentation (58).

8. Dispositif selon la revendication 7, dans lequel l'alimentation (58) est une alimentation électrique, et le moteur (22) est un moteur électrique.

9. Dispositif selon la revendication 8, dans lequel l'alimentation électrique comprend une batterie.

10. Dispositif selon la revendication 1, comprenant en outre un moteur implantable (22) pour faire fonctionner le moyen d'ajustement (12), ledit moyen pour le transfert d'énergie sans fil étant apte à alimenter directement le moteur en énergie transférée.

11. Dispositif selon la revendication 10, dans lequel l'énergie transférée par ledit moyen de transfert d'énergie comprend des signaux d'onde.

12. Dispositif selon la revendication 10, dans lequel l'énergie transférée par ledit moyen de transfert d'énergie comprend un champ électrique ou un champ magnétique.

13. Dispositif selon la revendication 1, dans lequel le moyen de commande à distance (22, 26, 32-44) est apte à obtenir des informations se rapportant à la taille de l'ouverture de restriction (3) et à commander au moyen d'ajustement (12) d'ajuster l'élément de restriction (2) en réponse aux informations obtenues.
